# EUROPEAN PATENT APPLICATION

(11) **EP 3 689 376 A1**
(43) Date of publication of application: **05.08.2020**
(21) Application number: 18863457.0
(22) Date of filing: 26.09.2018
(51) Int. Cl.: A61K 39/395, A61K 31/4745, A61P 35/00, A61P 35/02, A61P 43/00

(54) **PHARMACEUTICAL COMPOSITION**

(30) Priority: 26.09.2017 JP 2017184968
(71) Applicant: Chugai Seiyaku Kabushiki Kaisha, Kita-ku Tokyo 115-8543 (JP)
(72) Inventor: IWAI, Toshiki, Kamakura-shi Kanagawa 247-8530 (JP); YAMAMOTO, Kaname, Kamakura-shi Kanagawa 247-8530 (JP); SUGIMOTO, Masamichi, Kamakura-shi Kanagawa 247-8530 (JP)
(74) Representative: Vossius & Partner Patentanwälte Rechtsanwälte mbB
(86) International application number: PCT/JP2018/035655
(87) International publication number: WO 2019/065720

(57) **Abstract**

The present invention relates to a pharmaceutical composition comprising an anti-PD-Ll antibody as an active ingredient, wherein the pharmaceutical composition is used in combination with camptothecin and/or a derivative thereof, or a pharmaceutically acceptable salt thereof.

## Description

### Technical Field

The present invention relates to a pharmaceutical composition comprising an anti-PD-L1 antibody as an active ingredient, wherein the pharmaceutical composition is used in combination with camptothecin and/or a derivative thereof, or a pharmaceutically acceptable salt thereof.

### Background Art

Immune checkpoint acts to suppress excessive immune reaction so as to inhibit the onset of autoimmune diseases and the like. As immune checkpoint proteins involved in this mechanism, PD-1 and the like on T cells are known, which respond to a PD-L1 ligand on the surface of tumor cells, immune cells or the like. An example of antibodies against these proteins, a PD-L1 antibody, is an immune checkpoint inhibitor. Through administration of such an immune checkpoint inhibitor, T-cell immunosuppression is ceased and the antitumor immune response is enhanced.

Camptothecin is a cytotoxic quinoline alkaloid and is a compound having anticancer activity, which is isolated from the bark and trunk of drought lotus tree (*Camptotheca acuminata*). Camptothecin has excellent anticancer actions, but is slightly soluble and has harmful side effects, and thus derivatives thereof have been studied to address these problems. Known derivatives of camptothecin obtained by improvement thereof include irinotecan (CPT-11), nogitecan (JAN) and topotecan (INN)). These derivatives have a topoisomerase I-inhibiting action. An irinotecan hydrochloride hydrate is commercially available in the trade name of CAMPTO for I.V. infusion (R) or Topotecin (R), and a nogitecan hydrochloride is commercially available in the trade name of Hycamtin (R).

A combined use of immunotherapy has been studied using an immune checkpoint inhibitor and chemotherapy using an anticancer agent. It has been recently reported that through the use of MM-398 (ONIVYDE (R), irinotecan·liposome injection) that is a liposomal preparation of irinotecan in combination with a mouse anti-PD-1 or an anti-PD-L1 monoclonal antibody exhibits an effect on a mouse colon cancer cell transplantation model stronger than that exhibited by each single agent (Patent Literature 1).

However, a more effective therapy using a synergistic effect of the combined use of an anticancer agent and immunotherapy is still under exploration.

### Citation List

### Patent Literature

Patent Literature 1: WO 2017/049199

### Summary of Invention

### Technical Problem

The present invention provides a combined use of a novel anticancer agent and immunotherapy.

### Solution to Problem

As a result of intensive studies, the present inventors have discovered that a combination of an anti-PD-Ll antibody and irinotecan exhibits a synergistic effect, and thus have completed the present invention.

The present invention encompasses the following aspects.
[1] A pharmaceutical composition comprising an anti-PD-L1 antibody as an active ingredient, wherein the pharmaceutical composition is used in combination with camptothecin and/or a derivative thereof, or a pharmaceutically acceptable salt thereof.
[2] A pharmaceutical composition comprising camptothecin and/or a derivative thereof, or a pharmaceutically acceptable salt thereof as an active ingredient, wherein the pharmaceutical composition is used in combination with an anti-PD-Ll antibody.
[3] A pharmaceutical composition containing camptothecin and/or a derivative thereof, or a pharmaceutically acceptable salt thereof, and an anti-PD-Ll antibody.
   [3-1] The pharmaceutical composition according to any one of [1] to [3], wherein camptothecin and/or a derivative thereof, or a pharmaceutically acceptable salt thereof is a hydrochloride of camptothecin.
[4] The pharmaceutical composition according to any one of [1] to [4], wherein the camptothecin derivative is irinotecan.
   [4-1] The pharmaceutical composition according to any one of [1] to [4], wherein the camptothecin derivative is irinotecan hydrochloride or nogitecan hydrochloride.
   [4-2] The pharmaceutical composition according to any one of [1] to [4], wherein the camptothecin derivative is an irinotecan hydrochloride intravenous infusion solution or a nogitecan hydrochloride intravenous infusion solution.
[5] The pharmaceutical composition according to any one of [1] to [4], wherein the pharmaceutical composition is an agent for treating cancer.
[6] The pharmaceutical composition according to [5], wherein the cancer is selected from the group consisting of breast cancer, liver cancer, lung cancer including small cell lung cancer, non-small-cell lung cancer, ovarian cancer, gastric cancer, bladder cancer, pancreatic cancer, endometrial cancer, cervical cancer, colon·rectal cancer, melanoma, squamous cell carcinoma, Merkel cell carcinoma, pediatric malignant solid tumor, glioma, thyroid cancer, urothelial cancer, head and neck cancer, renal cancer, esophageal cancer, prostate cancer, malignant lymphoma and leukemia.
   [6-1] The pharmaceutical composition according to [5], wherein the cancer is gastric cancer, rectal/colon cancer, small cell lung cancer, non-small-cell lung cancer, bladder cancer, renal cancer, liver cancer, urothelial cancer, cervical cancer, head and neck cancer, esophageal cancer or breast cancer.
[7] The pharmaceutical composition according to [5], wherein the cancer is small cell lung cancer or breast cancer.
[8] The pharmaceutical composition according to any one of [1] to [7], wherein the anti-PD-Ll antibody is Atezolizumab, Avelumab, Durvalumab, KN035, CX-072, LY3300054, and/or FAZ053.
   [8-1] The pharmaceutical composition according to any one of [1] to [7], wherein the anti-PD-Ll antibody is Atezolizumab, Avelumab or Durvalumab.
   [8-2] The pharmaceutical composition according to any one of [1] to [7], wherein the camptothecin derivative is irinotecan hydrochloride or nogitecan hydrochloride, and the anti-PD-Ll antibody is Atezolizumab, Avelumab, Durvalumab, KN035, CX-072, LY3300054, and/or FAZ053.
   [8-3] The pharmaceutical composition according to any one of [1] to [7], wherein the camptothecin derivative is irinotecan hydrochloride or nogitecan hydrochloride, and the anti-PD-Ll antibody is Atezolizumab, Avelumab or Durvalumab.
   [8-4] The pharmaceutical composition according to any one of [1] to [7], wherein the camptothecin derivative is irinotecan hydrochloride or nogitecan hydrochloride, and the anti-PD-Ll antibody is Atezolizumab.
[9] A method for treating cancer, comprising using an anti-PD-L1 antibody and camptothecin and/or a derivative thereof, or a pharmaceutically acceptable salt thereof in combination.
   [9-1] A method for treating cancer, wherein an anti-PD-Ll antibody and camptothecin and/or a derivative thereof, or a pharmaceutically acceptable salt thereof are Atezolizumab and irinotecan hydrochloride or nogitecan hydrochloride.

### Advantageous Effects of Invention

The present invention is useful for treating cancer.

### Brief Description of Drawings

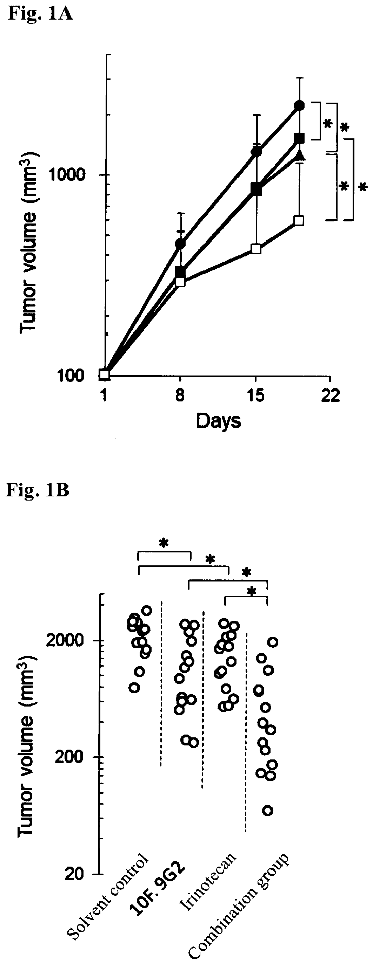

[Figure 1A] Figure 1A depicts changes in tumor volume of a mouse breast cancer cell line, FM3A, in each mouse subjected to the administration of a solvent control (●), monotherapy (10 mg/kg of 10F.9G2 (anti-PD-Ll antibody) administered 3 times a week (▲) or 250 mg/kg of irinotecan administered once at the initiation of administration (■)), or the administration of a combination (10F.9G2+irinotecan) (□). Fourteen (14) mice per group were treated. The average tumor volume and SD bar of each group were plotted. The vertical axis represents tumor volume (mm³) and the horizontal axis represents the number of days. * denotes p<0.05. [Figure 1B] Figure 1B depicts the individual tumor volumes of each group and a solvent control group on the last day of the test of Figure 1A (day 19 after the initiation of administration). The vertical axis represents tumor volume (mm³). The horizontal axis represents, from the left, the results of the solvent control, 10F.9G2 antibody, irinotecan, and a combination of 10F.9G2 antibody and irinotecan.

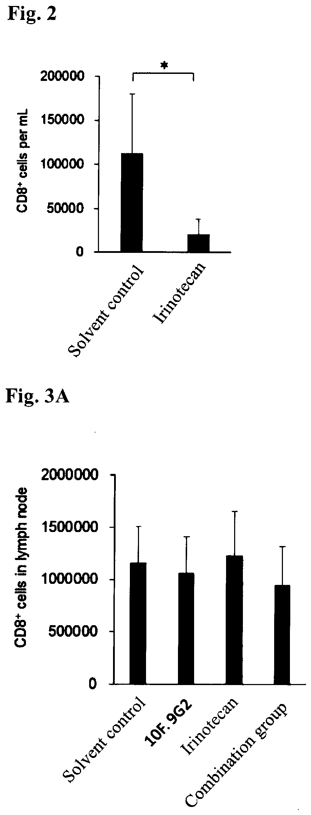

[Figure 2] Figure 2 depicts a graph obtained by plotting the numbers of CD8-positive T cells in the peripheral blood on day 8 in the solvent control group and 250 mg/kg of irinotecan alone-administered group. Six (6) mice per group were treated. The vertical axis represents the number of CD8-positive T cells per 1 ml. The horizontal axis represents, from the left, the results of the solvent control and irinotecan.
[Figure 3A] Figure 3A depicts the number of CD8-positive T cells among all the living cells in a tumor on day 8 after the initiation of administration in each group to which any one of a solvent control, 10 mg/kg of 10F.9G2, 250 mg/kg of irinotecan, and 250 mg/kg of a combination (10F.9G2+irinotecan) was administered. The horizontal axis represents, from the left, the results of the solvent control, the anti-PD-L1 antibody, irinotecan, and the combination of the anti-PD-Ll antibody and irinotecan.

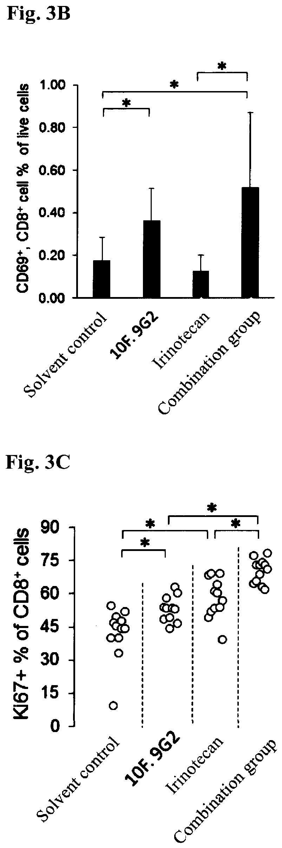

[Figure 3B] Figure 3B depicts the number of CD69-positive CD8-positive T cells among all the living cells in a tumor on day 8 after the initiation of administration in each group, to which any one of a solvent control, 10 mg/kg of 10F.9G2, 250 mg/kg of irinotecan, and 250 mg/kg of a combination (10F.9G2+irinotecan) was administered. Twelve (12) mice per group were treated. The vertical axis represents the number of cells. The horizontal axis represents, from the left, the results of the solvent control, the anti-PD-L1 antibody, irinotecan, and the combination of the anti-PD-L1 antibody and irinotecan.
[Figure 3C] Figure 3C is a graph obtained by plotting the proportion of Ki-67-positive cells among CD8-positive T cells in a tumor on day 8 after the initiation of administration in each group to which a solvent control, 10 mg/kg of 10F.9G2, 250 mg/kg of irinotecan, and 250 mg/kg of a combination (10F.9G2+irinotecan) was administered. Twelve (12) mice per group were treated. The vertical axis represents the number of cells. The horizontal axis represents, from the left, the results of the solvent control, the anti-10F.9G2 antibody, irinotecan, and the combination of 10F.9G2 and irinotecan.

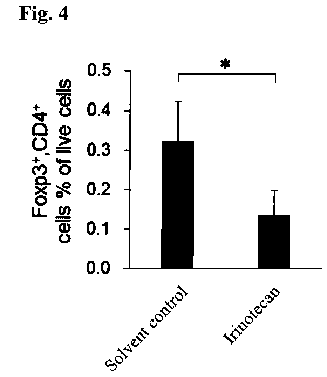

[Figure 4] Figure 4 is a graph obtained by plotting the numbers of Foxp3-positive CD4-positive regulatory T cells in the individual tumors on day 8 after the initiation of administration in the solvent control group and the 250 mg/kg of irinotecan alone-administered group. Twelve (12) mice per group were treated. The vertical axis represents the number of Foxp3-positive CD4-positive regulatory T cells, and the horizontal axis represents, from the left, the results of the solvent control and irinotecan.

### Description of Embodiments

Examples of camptothecin and/or a derivative thereof, or a pharmaceutically acceptable salt thereof include, preferably, irinotecan and/or nogitecan or a pharmaceutically acceptable salt thereof. For example, as irinotecan hydrochloride hydrate, those commercially available in the trade names, CAMPTO for I.V. infusion and Topotecin can be used, and as nogitecan hydrochloride, those commercially available in the trade name, Hycamtin, can be used.

The anti-PD-Ll antibody can be obtained by known means as a polyclonal antibody or a monoclonal antibody. The origin of such an antibody is not particularly limited, and the anti-PD-Ll antibody is preferably a mammal-derived antibody, and more preferably a human-derived antibody. Examples of a mammal-derived monoclonal antibody include those produced by hybridomas, and those produced by a host transformed by a genetic engineering technique with an expression vector containing an antibody gene.

The anti-PD-Ll antibody is an antibody that binds to PD-L1, preferably an antibody that binds to PD-L1 to inhibit the functions, further preferably an antibody that inhibits functions by inhibiting the binding of PD-1 or B7.1 with PD-L1, and more preferably an antibody that inhibits signals induced by binding of PD-1 or B7.1 with PD-L1.

The expression "inhibit the functions of PD-L1" means that the suppression of T cell activation induced by binding of PD-1 or B7.1 with PD-L1 is ceased. The PD-L1 activity is decreased by at least 5%, 10%, 20%, 30%, 40%, 50%, 60%, 70%, 80%, 90%, or 100% compared with the activity in a control. The PD-L1 activity is determined by any standard method in the art (examples thereof include those described herein).

As the anti-PD-Ll antibody, for example, Atezolizumab, Avelumab, Durvalumab, KN035, CX-072, LY3300054, and FAZ053 can be used.

An antibody to be used herein may be a conjugate antibody prepared by binding with various molecules such as polyethylene glycol (PEG), a radioactive substance, and toxin. Such a conjugate antibody can be obtained by chemically modifying the thus obtained antibody. Note that a method for modifying an antibody has already been established in the field. Examples of the term "antibody" used herein also include these conjugate antibodies.

Examples of the antibody include, not only divalent antibodies represented by IgG, but also monovalent antibodies, or polyvalent antibodies represented by IgM. Examples of the polyvalent antibody of the present invention include, polyvalent antibodies, all of which have the same antigen binding site, or, polyvalent antibodies, all of which have partially or completely different antigen binding sites.

Further, the antibody may be a bispecific antibody. A bispecific antibody has variable regions recognizing different epitopes in the same antibody molecule, wherein the epitopes may be present in different molecules or the same molecule.

A method for producing a bispecific antibody is known. For example, 2 types of antibodies that recognize different antigens are bound, so that a bispecific antibody can be prepared. Each antibody to be bound may be a half-molecule of an antibody having H and L chains, or a quarter-molecule of an antibody composed only of H chain. Alternatively, hybridomas producing different monoclonal antibodies are fused, so that a bispecific antibody-producing fused cell can also be prepared. Further, a bispecific antibody can be prepared by a genetic engineering technique.

The antibody may be a low molecular antibody. Examples of the low molecular antibody include an antibody fragment prepared by deleting a portion from a full-length antibody. As long as it binds to Arid5A, a partial deficiency in an antibody molecule is acceptable. An antibody fragment to be used in the present invention preferably contains either a heavy chain variable region (VH) or a light chain variable region (VL), or both VH and VL. The amino acid sequence of VH or VL can contain an addition, a deletion and/or a substitution. Further, as long as it binds to PD-L1, either VH or VL, or portions of both VH and VL can also be deleted. Moreover, the antibody fragment may be in a chimerized or humanized form. Specific examples of the antibody fragment can include Fab, Fab', F(ab')2, and Fv. Further, specific examples of the low molecular antibody can include Fab, Fab', F(ab')2, Fv, scFv, diabody, and sc(Fv)2.

Examples of pharmaceutically acceptable materials can include sterile water and physiological saline, stabilizers, excipients, buffering agents, antiseptics, surfactants, chelating agents (EDTA and the like), and binders.

Examples of a surfactant can include nonionic surfactants. Typical examples thereof can include those having HLB6-18: sorbitan fatty acid esters such as sorbitan monocaprylate, sorbitan monolaurate, and sorbitan monopalmitate; and glycerol fatty acid esters such as glycerol monocaprylate, glycerol monomyristate, and glycerol monostearate.

Examples of a surfactant can also include anionic surfactants. Typical examples thereof can include: alkyl sulfates each having an alkyl group having 10 to 18 carbon atoms, such as sodium acetyl sulfate, sodium lauryl sulfate, and sodium oleyl sulfate; polyoxyethylene alkyl ether sulfates each having an average number of moles added of an ethylene oxide ranging from 2 to 4 and having an alkyl group having 10 to 18 carbon atoms, such as sodium polyoxyethylene laurylsulfate; alkyl sulfosuccinate salts each having an alkyl group having 8 to 18 carbon atoms, such as a sodium lauryl sulfosuccinate; natural surfactants such as lecithin and glycerophospholipid; sphingophospholipids such as sphingomyelin; and sucrose fatty acid esters each having a fatty acid having 12 to 18 carbon atoms.

Examples of a buffering agent can include phosphoric acid, a citrate buffer, acetic acid, malic acid, tartaric acid, succinic acid, lactic acid, potassium phosphate, gluconic acid, caprylic acid, deoxycholic acid, salicylic acid, triethanolamine, and fumaric acid, other organic acids, and a carbonate buffer, a tris buffer, a histidine buffer, and an imidazole buffer.

Further, a solution preparation may also be prepared by dissolving in an aqueous buffer known in the field of solution preparation. The concentration of a buffer ranges from generally 1 to 500 mM, preferably 5 to 100 mM, and further preferably 10 to 20 mM.

Examples of saccharides such as a polysaccharide and a monosaccharide and carbohydrates can include dextran, glucose, fructose, lactose, xylose, mannose, maltose, sucrose, and raffinose.

Examples of sugar alcohol can include mannitol, sorbitol, and inositol.

When an aqueous solution for injection is prepared, examples thereof include physiological saline and an isotonic solution containing dextrose and another adjuvant, such as D-sorbitol, D-mannose, D-mannitol, and sodium chloride, which may be used in combination with appropriate solubilizing agents, such as alcohol (e.g., ethanol), polyalcohol (e.g., propylene glycol and PEG), a nonionic surfactant (polysorbate 80, HCO-50), and the like.

If desired, a diluent, a solubilizing agent, a pH adjusting agent, a soothing agent, a sulfur-containing reducing agent, an antioxidant, and the like may also be contained.

One aspect of the present invention is a pharmaceutical composition comprising an anti-PD-Ll antibody as an active ingredient, wherein the pharmaceutical composition is used in combination with camptothecin and/or a derivative thereof, or a pharmaceutically acceptable salt thereof.

One aspect of the present invention is a pharmaceutical composition comprising camptothecin and/or a derivative thereof, or a pharmaceutically acceptable salt thereof as an active ingredient, wherein the pharmaceutical composition is used in combination with an anti-PD-Ll antibody.

One aspect of the present invention is a pharmaceutical composition containing camptothecin and/or a derivative thereof, or a pharmaceutically acceptable salt thereof and an anti-PD-L1 antibody.

One aspect of the present invention is a medicament comprising a combination of camptothecin and/or a derivative thereof, or a pharmaceutically acceptable salt thereof and an anti-PD-Ll antibody.

The pharmaceutical composition of the present invention can be used for treating cancer. The cancer is selected from the group consisting of breast cancer, liver cancer, lung cancer including small cell lung cancer, ovarian cancer, gastric cancer, bladder cancer, pancreatic cancer, endometrial cancer, cervical cancer, colon·rectal cancer, renal cancer, esophageal cancer, prostate cancer, melanoma, squamous cell carcinoma, Merkel cell carcinoma, pediatric malignant solid tumor, glioma, thyroid cancer, urothelial cancer, head and neck cancer, malignant lymphoma and leukemia. Preferably, the cancer is gastric cancer, rectal/colon cancer, small cell lung cancer, non-small-cell lung cancer, bladder cancer, renal cancer, liver cancer, urothelial cancer, cervical cancer, head and neck cancer, esophageal cancer or breast cancer.

A medicament according to the present invention can be prepared by formulating these active ingredients into a single preparation (compounding agent) or separately formulating the active ingredients into two or more types of preparations. The above preparations can be prepared by generally employed means in the form of tablets, granules, powders, capsules, emulsions, suspensions, or syrups, or, injections such as aseptic solutions, and suspensions. When these active ingredients are separately formulated into two or more types of preparations, individual preparations can be administered simultaneously or separately at given time intervals. The two or more types of preparations can also be separately administered in different frequencies daily. The medicament according to the present invention can be administered systemically or topically via peroral administration or parenteral administration. When these active ingredients are separately formulated into two or more types of preparations, individual preparations can be administered via different routes of administration.

When the medicament according to the present invention is prepared in the form of two different types of preparations, since these preparations are likely administered simultaneously or at extremely short intervals, for example, documents such as those attached to commercially available remedies and sales pamphlets can describe a combined use of these preparations. Further, the medicament can also be provided in the form of a kit comprising these preparations.

The dosage of the medicament of the present invention differs depending on administration subjects, administration methods, and the like. For example, the dosage of irinotecan in the form of irinotecan hydrochloride hydrate is, via IV infusion, 10 mg/m² or more, 20mg/m² or more, 40mg/m² or more, 100mg/m² or more, 150mg/m² or more, or 180 mg/m² or more once daily, and thus irinotecan can be administered in a dosage of 1,000 mg/m² or less. The dosage of nogitecan in the form of nogitecan hydrochloride is, via IV infusion, 0.75 mg/m² or more, 1.0 mg/m² or more, or 1.5 mg/m² or more once daily, and thus nogitecan can be administered in a dosage of 1,000 mg/m² or less. Further, the dosage of the anti-PD-Ll antibody is, for example, 1 mg or more per administration, 840 mg or less, 1,000 mg or less, or 1,200 mg or less per administration, or the anti-PD-Ll antibody can be administered in a dosage of 1.0 mg/kg (body weight) or more, 2.5 mg/kg (body weight) or more, 10 mg/kg (body weight) or more, or 20 mg/kg (body weight) or more per administration, and thus the anti-PD-Ll antibody can be administered in a dosage of 100 mg/kg (body weight) or less.

One aspect of the present invention is a method for treating or preventing cancer, comprising using an anti-PD-Ll antibody and camptothecin and/or a derivative thereof, or a pharmaceutically acceptable salt thereof in combination. The anti-PD-L1 antibody and camptothecin and/or a derivative thereof, or a pharmaceutically acceptable salt thereof can be administered simultaneously or sequentially to a patient.

In one aspect of the present invention, examples of the above-intended "used in combination (with)" include, simultaneous administration of different preparations or individual medicament preparations, and, preferably sequential administration in an arbitrary order, which allows a sufficient time for both (or all) active agents to simultaneously exhibit biological activity. Formulation and medication schedules for such chemotherapeutics can be used according to manufacturers' instructions or can be empirically determined and used by persons skilled in the art.

### Examples

### Example 1

Hereinafter, the present invention will be described more specifically with reference to Examples, but the invention is not limited to these Examples.

### Antitumor activity in the case of combined use of anti-PD-L1 antibody (10F.9G2) and irinotecan in syngeneic mouse model using mouse breast cancer cell line FM3A

FM3A cells were cultured using cell culture flasks in an incubator (set at 37°C and 5% CO₂). Cells were collected, re-suspended at 1×10⁷ cells/mL, and then subcutaneously inoculated into the right abdomen of each C3H/HeN mouse (Charles River Laboratories Japan) (1×10⁶ cells/mouse). Once palpable tumors were established, the relevant animals were randomized into test groups in such a manner that each group had a similar average tumor volume at the initiation of the test. The day of randomization was designated as the day of initiating drug administration, and then 250 mg/kg of irinotecan was administered intraperitoneally once, and 10 mg/kg of 10F.9G2 (purchased from BioLegend, Inc.) was administered intraperitoneally 3 times a week. Physiological saline was administered as a solvent control relative to irinotecan, and rat IgG was administered as a negative control relative to the anti-PD-L1 antibody. When the average tumor volume of the solvent control group on the last day of the test was determined to be 1.0, the average tumor volume upon administration of 10 mg/kg of 10F.9G2 alone and that of 250 mg/kg of irinotecan alone were 0.58 and 0.69, respectively. The average tumor volume upon administration of the combination group (10F.9G2+irinotecan), 0.27, was lower than the product of the two average tumor volumes (0.58 x 0.69), 0.40. Hence, the combination was evaluated as Supra-Additive based on the Interaction Index for the effects of combination (Figure 1 and Table 1).

**[Table 1]**

| | Anti-PD-L1 antibody | CPT-11 | Combination |
|---|---|---|---|
| Tumor growth ratio | 0.58 | 0.69 | 0.27 |
| Additive theoretical value | - | - | 0.40 |

Tumor growth ratio: Rate of change in tumor volume of treatment group/Rate of change in tumor volume of control group
Rate of change in tumor volume: Tumor volume on the last day of measurement/Tumor volume on the day of initiation of administration Additive theoretical value: Product of tumor growth ratios of single agents

### Example 2

### Effects of irinotecan on the number of CD8-positive T cells in peripheral blood in syngeneic mouse model using mouse breast cancer cell line FM3A

FM3A cancer-bearing mice were prepared in the same manner as described above, and then on the day of initiating administration, 250 mg/kg of irinotecan was administered intraperitoneally once. On day 8 of administration, peripheral blood was collected, CD8-positive T cells were measured by flow cytometry (Figure 2). The irinotecan administration group was found to have the number of CD8-positive T cells in peripheral blood significantly lower (P<0.05, Wilcoxon Signed-Rank Test, the same applies to the following) than that of the solvent administration group.

### Example 3

### Effects of the administration of PD-L1 antibody alone, the administration of irinotecan alone, and the administration of a combination of the two on CD8-positive T cells in tumor in syngeneic mouse model using mouse breast cancer cell line FM3A

FM3A cancer-bearing mice were prepared in the same manner as described above, and on the day of initiating administration, 250 mg/kg of irinotecan was administered intraperitoneally once, and 10 mg/kg of 10F.9G2 was administered intraperitoneally 3 times a week. On day 8 of administration, tumors were collected, and then cells were dissociated using a gentleMACS (Trademark) Octo Dissociator, washed, and then used for flow cytometry, thereby quantitatively determining CD8, CD69 or Ki-67-positive tumor-infiltrating lymphocytes (TIL) (Figure 3). The irinotecan administration group was not found to exhibit any significant decrease in CD8-positive T cells in lymph nodes, and CD69-positive CD8-positive T cells among tumor cells, compared with the solvent administration group (Figure 3A and Figure 3B). The irinotecan administration group and the 10F.9G2 group were found to exhibit significant increases in Ki-67-positive CD8-positive T cells in tumors compared with the solvent administration group. The combination group was found to exhibit a significant increase in the same compared with each single agent administration group (Figure 3C).

### Effects of irinotecan on Foxp3-positive CD4-positive regulatory T cells in tumor in syngeneic mouse model using mouse breast cancer cell line FM3A

FM3A cancer-bearing mice were prepared in the same manner as described above, and on the day of initiating administration, 250 mg/kg of irinotecan was administered intraperitoneally once. On day 8 of administration, tumors were collected, and then cells were dissociated using a gentleMACS (Trademark) Octo Dissociator, washed, and then used for flow cytometry, thereby measuring Foxp3-positive CD4-positive regulatory T cells by flow cytometry (Figure 4). The irinotecan administration group was found to have Foxp3-positive CD4-positive regulatory T cells in tumors significantly lower than those of the solvent administration group.

### Industrial Applicability

The present invention is useful for treating cancer.

## Claims

1. A pharmaceutical composition comprising an anti-PD-Ll antibody as an active ingredient, wherein the pharmaceutical composition is used in combination with camptothecin and/or a derivative thereof, or a pharmaceutically acceptable salt thereof.

2. A pharmaceutical composition comprising camptothecin and/or a derivative thereof, or a pharmaceutically acceptable salt thereof as an active ingredient, wherein the pharmaceutical composition is used in combination with an anti-PD-Ll antibody.

3. A pharmaceutical composition containing camptothecin and/or a derivative thereof, or a pharmaceutically acceptable salt thereof, and an anti-PD-Ll antibody.

4. The pharmaceutical composition according to any one of claims 1 to 3, wherein the derivative of camptothecin is irinotecan.

5. The pharmaceutical composition according to any one of claims 1 to 4, wherein the pharmaceutical composition is an agent for treating cancer.

6. The pharmaceutical composition according to claim 5, wherein the cancer is selected from the group consisting of breast cancer, liver cancer, lung cancer including small cell lung cancer, non-small-cell lung cancer, ovarian cancer, gastric cancer, bladder cancer, pancreatic cancer, endometrial cancer, cervical cancer, colon·rectal cancer, melanoma, squamous cell carcinoma, Merkel cell carcinoma, pediatric malignant solid tumor, glioma, thyroid cancer, urothelial cancer, head and neck cancer, renal cancer, esophageal cancer, prostate cancer, malignant lymphoma and leukemia.

7. The pharmaceutical composition according to claim 5, wherein the cancer is small cell lung cancer or breast cancer.

8. The pharmaceutical composition according to any one of claims 1 to 6, wherein the anti-PD-Ll antibody is Atezolizumab, Avelumab, Durvalumab, KN035, CX-072, LY3300054, and/or FAZ053.

9. A method for treating cancer, comprising using an anti-PD-Ll antibody and camptothecin and/or a derivative thereof, or a pharmaceutically acceptable salt thereof in combination.
